# EUROPEAN PATENT SPECIFICATION

(11) **EP 3 097 750 B1**
(45) Date of publication and mention of the grant of the patent: **27.06.2018**
(21) Application number: 15708209.0
(22) Date of filing: 05.03.2015
(51) Int. Cl.: H05H 1/24, A61L 9/22

(54) **AIR DISINFECTION AND POLLUTION REMOVAL APPARATUS**
LUFTDESINFEKTIONS- UND VERSCHMUTZUNGSBESEITIGUNGSVORRICHTUNG
APPAREIL DE DÉSINFECTION D'AIR ET D'ÉLIMINATION DE POLLUTION

(30) Priority: 05.03.2014 US 201461948266 P; 10.03.2014 GB 201404185
(43) Date of publication of application: 30.11.2016
(73) Proprietor: Novaerus Patents Limited, Dublin (IE)
(72) Inventor: DEANE, Graham, Blackrock Co. Dublin (IE); MAUGHAN, Kevin, Blackrock Co. Dublin (IE); SOBERON, Felipe, Blackrock Co. Dublin (IE)
(74) Representative: Hanna Moore + Curley
(86) International application number: PCT/EP2015/054691
(87) International publication number: WO 2015/132368

(56) References cited:
- WO-A1-2011/123512
- WO-A1-2013/076459
- US-A1- 2006 182 672
- US-A1- 2007 253 860
- US-A1- 2012 093 691

## Description

### FIELD OF INVENTION

The present invention relates to an air treatment method and apparatus. More specifically, the invention relates to a method and apparatus for inactivating pathogens present in indoor air and removing pollutants from the same. Within the context of the present teaching there is provided a method and apparatus that effects an exposure of air to an atmospheric plasma discharge. This exposure results in a killing of bacteria, viruses and other pathogens in the air. The plasma discharge is also responsible for the dissociation of other pollutants present in the air.

### BACKGROUND OF THE INVENTION

Health threatening airborne pollutants may be subdivided into three groups; (a) airborne pathogens comprising any organism that causes disease that spreads throughout the environment via the air; (b) airborne allergens comprising any substance that, when ingested, inhaled, or touched, causes an allergic reaction and, (c) airborne volatile organic compounds (VOC) comprising any product that is designed to be sprayed at high pressure in the form of tiny particles that remain suspended in the air. The last category includes many cleaning chemicals, hair spray, various types of primer, and fuels such as gasoline and kerosene, as well as other household, beauty, or hobby products. Some fabrics, particularly those recently manufactured, also contribute to indoor airborne VOCs when they outgas, or leak out chemicals in gaseous form, over time.

Airborne pollutants can build up significantly in indoor environments with the result that the air that we breathe may become contaminated. Considering that on average humans spend approximately 90% of their time in an indoor environment, it will be appreciated that the removal of pollutants from indoor air is of importance to reduce allergies and prevent infection transmission, such as sick building syndrome.

Airborne pollutant control and removal is typically carried out in heating, ventilation and air conditioning (HVAC) systems by means of filter mediums. Current state of the art HVAC system filters capture particles ranging from a fraction of a micrometre (um) in diameter. High Efficiency Particulate Air (HEPA) filters are defined (by the US Department of Energy) as those that can capture 99.97% of particles with 0.3um diameter. These particles are the most penetrating particle size and the filter efficiency is higher for bigger particles. HEPA filters typically consist of randomly arranged fibreglass fibres with diameters between 0.2 and 0.5 um. Particles are trapped by the fibres by one of three mechanisms: interception, impaction and diffusion. Still, it is appreciated that these filters do not kill micro-organisms which in time may build up and grow on the filter becoming an actual infection reservoir that can spread the diseases throughout the ventilation system. It is also appreciated that using HEPA filter creates large pressure drops in the HVAC system reducing the overall performance of the ventilation system.

Existing state of the art technologies for the control of airborne pathogens can be categorized as: (a) airborne trapping systems or filters, (b) airborne inactivation systems and, (c) some combination of the above.

Airborne trapping systems include HEPA or similar filters, as described above with said limitations. Alternatively, electrostatic precipitators, such as that described in US patent publication No. 2013/0233172, are used to remove particles from the air. The efficiency of these devices varies with the particle size. In particular, these are known to exhibit poor performance for particles one micron or less in size, allowing airborne bacteria and virus to flow through. Furthermore, electrostatic precipitators tend to build dust on the collection plates reducing its performance over time.

Existing airborne inactivation technologies also include those that make use of chemicals, UV radiation and plasma discharge by-products.

Examples of chemical inactivation include the use of antimicrobial vaporizers, typically ozone or hydrogen peroxide. While these systems are effective, they are also disruptive, requiring the evacuation of indoor space to be treated and therefore are not suitable for use under normal living circumstances.

Alternative inventions for the purification of air comprise the use of ultra violet light (UV) emission to kill airborne bacteria. For example, international publication No. WO 2003/092751, describes a device in which a fluid (e.g. air) is passed through an array of UV lamps. It is appreciated that in this solution the one and only inactivation mechanism is via UV radiation.

Prior art also includes the use of plasma radicals for sterilisation of air filter medium; see for example US patent publication No. 2004/0184972 A1. In this document, it is proposed that an upstream plasma discharge can generate active radicals which flow upstream to a medium filter and kill any bacteria or virus trapped by the filter. The filters only capture particles potentially allowing bacteria population to grow over them. The disclosure of this document is such that the plasma radicals kill virus and bacteria trapped by the filter. This document provides, in addition, for a radical destruction filter for the destruction of excess radicals after the filter medium. These radicals are toxic to humans. It is appreciated that failures of said filter may result in release of toxic gases into living

Alternative treatments of polluted air by means of plasma generating assemblies are disclosed in US2006/0182672 A1 and US2007/0253860 A1. In summary, current state of the art for air disinfection and pollutant removal rely on either; air trapping devices which result in the accumulation pathogens which affect device performance and becomes a reservoir for infectious diseases or, air inactivation systems which rely on hazardous substances that pose severe risks to human health. It is therefore appreciated that an air treatment solution without the use of filter mediums or equivalent is required. Furthermore, such solution shall not include the generation of hazardous chemical substances.

The invention disclosed in the present document offers a viable solution to air disinfection and pollution control.

### SUMMARY OF THE INVENTION

Accordingly, a first embodiment of the application provides an air treatment apparatus as detailed in claim 1. Advantageous embodiments are provided in the dependent claims.

### BRIEF DESCRIPTION OF THE DRAWINGS

The invention will be more clearly understood from the following description of an embodiment thereof, given by way of example only, with reference to the accompanying drawings, in which:
Figure 1 is view of an embodiment of the apparatus of the present invention;
Figure 2 is view of the embodiment of Figure 1 displaying internal components of the apparatus of figure 1;
Figure 3 is a view of the apparatus of Figure 1 displaying internal components and the flow of air through said apparatus;
Figure 4 is a is a view of the electrode assembly of the apparatus of the present invention; and
Figure 5 is an exploded view of the electrode assembly of figure 4.

### DETAILED DESCRIPTION OF THE INVENTION

The present teaching relates to a method and apparatus for the removal of VOCs and inactivation of pathogens including but not limited to bacteria, virus, fungi and mould.

Within the context of the present teaching a method is provided comprising exposing a volume of air to an atmospheric plasma discharge for a given period of time. Said air volume is streamed through an apparatus containing the atmospheric plasma discharge. The apparatus serves as a housing for the plasma discharge and acts an air duct through which the air volume is streamed. The air is forced through the apparatus housing by an impeller or equivalent air forcing device, e.g. fan. The apparatus comprises of inlet and outlet regions to allow the air flow through the same. The plasma discharge is generated by applying high voltage to a pair set of electrodes. The applied high voltage is generated by suitable power supply able to sustain either a DC or an AC discharge between, around and/or on the surface of said electrode pair.

Pathogen inactivation and VOC removal is due to interaction of various species and radiation emanating from the plasma discharge with airborne pathogens. Plasma species include, but are not limited to, electrons, ions, and reactive radicals generated by the discharge. In addition the plasma discharge generates electric fields and ultraviolet radiation. The inactivation efficacy of said plasma discharge is proportional to the energy dissipated by the plasma discharge onto a volume of air flowing in the vicinity of the discharge. This energy is proportional to the power applied to sustain the plasma discharge and the exposure time period. The plasma exposure time of a given volume of air flowing through the apparatus is inversely proportional to the air flow.

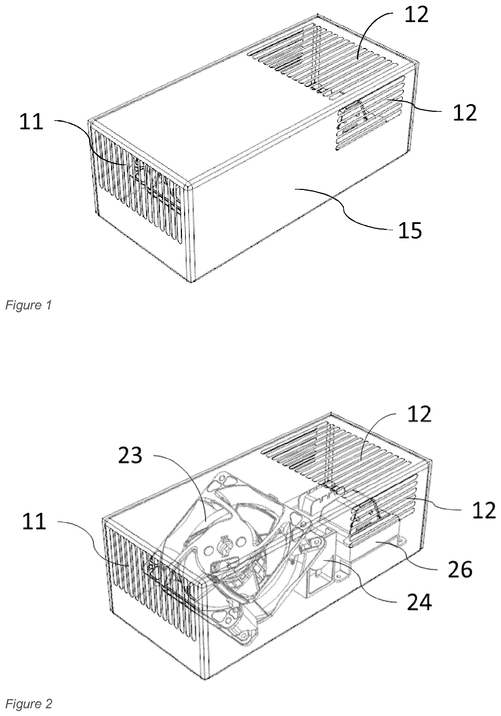

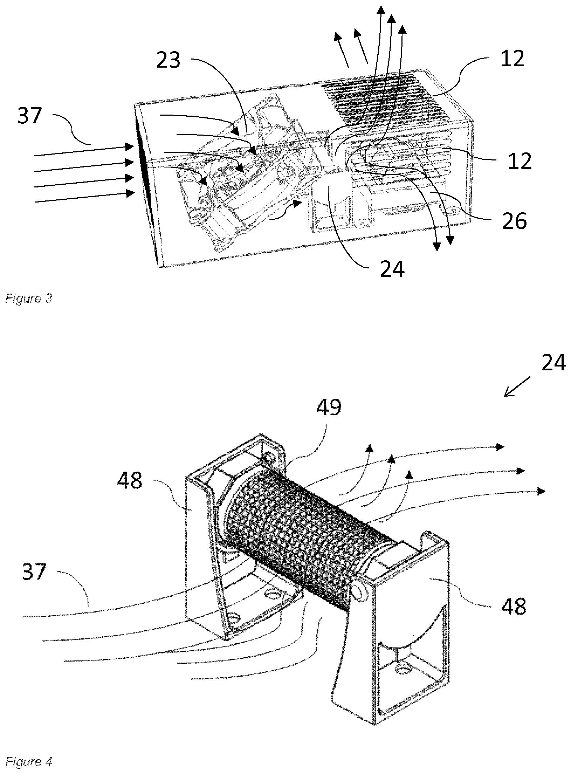

Thus, it is appreciated that the efficacy of the apparatus described here depends on two basic parameters: the plasma discharge power and the air flow. Those skilled in the art will find that there are a range of power and flow rates that meet various degrees of inactivation while meeting other criteria for the air treatment system, including the number of air changes per hour in a given room size and maximum allowed concentration of radicals generated by the plasma discharge. The present teaching may be further understood with reference to an example embodiment of the apparatus. Figure 1 shows a 3D projection of the apparatus consisting of a housing 15 and air inlet 11 and outlet 12 regions. These regions consist of opening or ventilation grids on the housing of the apparatus 15. Moreover, the apparatus contains within its housing an impeller 23, a set of electrodes 24 and a high voltage supply 26; as indicated in figure 2. Figure 3 further illustrates the flow of air induced by the impeller 23. Air 37 is forced into the apparatus via the inlet region, forced around the electrode set assembly 24 and expelled through the outlet regions 12. The high voltage supply 26, for example a high voltage transformer, sustains a plasma discharge across electrode assembly 24. The electrode configuration and construction can be chosen among a variety of electrode arrangements for atmospheric plasma discharges. Those skilled in the art will find more than one design may fit the apparatus and method described here. For instance, a pair of concentric wire mesh cylinders separated by a cylindrical dielectric barrier, made of ceramic, glass or other suitable material, will suffice.

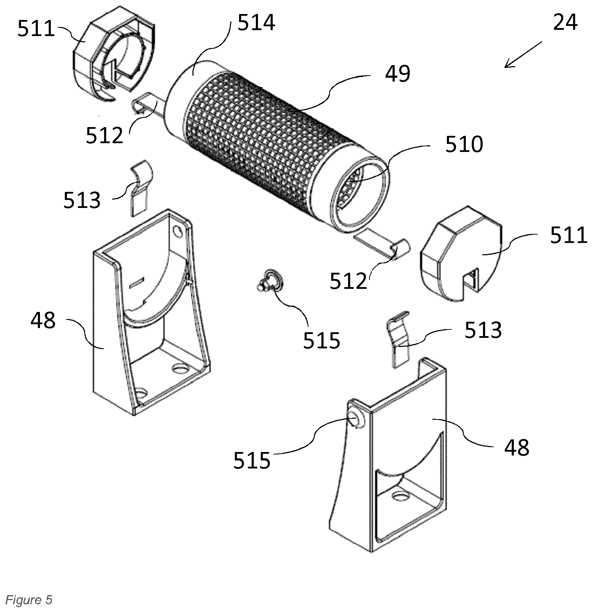

A preferred embodiment for the electrode assembly and support is shown in Figure 4. This figure further illustrates the flow of air 37 around the surface of electrode assembly 24. In this embodiment, the electrode assembly 24 is shown with an outer wire mesh cylinder 49 and the assembly 24 includes mounting holders 48. The plasma mode in this embodiment is of a dielectric barrier discharge (DBD) type with an inner wire mesh cylinder insulated by a dielectric glass tube as will be explained with reference to figure 5. The cylindrical shape of the electrode assembly ensures that the outer mesh extends completely circumferentially around the electrode assembly and that plasma is discharged evenly in all directions from the electrode assembly.

It will be appreciated that the voltage and current parameters required to achieve a dielectric barrier discharge will depend principally on the nature of the dielectric used, as further discussed herein below. In general operating voltages below 1 kV are not practical, and preferably there is used an operating voltage in the range from 1 to 6 kV, most desirably from 3 to 5 kV, for example about 4 kV. It will be appreciated that the current required to maintain the dielectric barrier discharge is significantly less than that required to initiate it. The current (and hence power) of plasma generator units is normally expressed in terms of the starting current. There should be used a (starting) current in the range from 1 to 10 mA, preferably at least 3 mA. The power of the unit will of course depend on the voltage and current combination. The power should generally be not more than 50 watts, and is preferably at least 4 watts. Typically the power is in the range from 10 to 40 watts. These power levels have in particular been found to be convenient with an apparatus unit having a conduit volume of the order of 0.02 to 1.0 m³ Even with such low power dielectric barrier discharge units it has been found possible to achieve well contained localized highly inactivating concentrations of anti-pathogenic agents sufficient to inactivate a very wide range of airborne pollutants or pathogens as is evidenced from the data in Table 1 and Table 2 below.

Advantageously the plasma generator is coupled to a transformer which is provided with an anti-surge and/or anti-spike device(s), in order to minimize transient excursions of the output voltage above the normal level which could result in temporary extension of the inactivation zone outside of the housing within which the electrodes are provided and/or generation of excessively high anti-pathogenic agent levels.

Importantly, the transformer is located outside of the direct air flow path to minimize the risk of exposure to plasma/anti-pathogenic agents and possible breakdown in the course of use of the apparatus. As previously mentioned, this also ensures that the transformer does not interfere with the flow of air passed the electrode assembly.

An AC supply may also be provided in the apparatus. Preferably, this is co-located with the transformer such that it too does not obstruct airflow. A wide range of frequencies may be used in the AC supply to the low power dielectric barrier discharge device, and indeed somewhat higher frequencies may safely be used than is possible with conventional high power plasma generators. Conveniently an AC supply with a frequency in the range from 50 to 1000 Hz may be used.

It will be appreciated that the sizing and placement of the electrodes that define the discharge unit with respect to the airflow path is quite important. The conduit within which they are located should not be smaller than a volume required to contain the inactivation zone of the plasma generated by the dielectric barrier discharge and not so large that substantially the whole of the airflow within the housing does not pass through said inactivating zone in the course of its transit through apparatus. It will be appreciated that in this context, the inactivating zone is a volume surrounding the discharge unit containing an elevated concentration of anti-pathogenic agents, sufficient to substantially inactivate airborne pollutants or pathogens. The inactivating zone is situated on the outer surface of the discharge unit and in a downstream direction. The total inactivating zone is the combination of all upstream, mid, and downstream stages as anti-pathogenic agents tend to disperse in whole volume and cause deactivation of micro-organisms.

Airflow is desirably passed over and under the electrodes of the discharge unit and not passed through it i.e., there are no air gaps within the discharge unit that allow air to enter or that could potentially hold a build-up in anti-pathogenic agents.

In accordance with the present teaching a discharge unit is provided that is there is desirably used with a solid dielectric to provide a dielectric barrier discharge which the present inventors have found provides in order to obtain a more consistent and reliable plasma generation performance. Various geometries are also possible. However, for the present application, a generally tubular geometry, with a tubular dielectric with generally tubular electrodes on the inner and outer faces thereof is preferred. This tubular shape works to direct the airflow around the discharge unit. It will be appreciated that plasma will be generated at both electrodes. Preferably there is used a generally mesh form electrode, providing a coil, in order to maximize the areas of dielectric surface at which plasma is generated. In this connection it will be appreciated that substantially "closed" meshes are less desirable as these reduce the exposed dielectric surface. On the other hand excessively "open" meshes are generally less efficient in the amount of plasma generated for a give size of unit. Most preferably the electrodes are perfectly coaxial cylindrical mesh where the dielectric is sandwiched between them. In this context preferably the low power discharge unit comprises concentric tubular metal gauze electrodes separated by a dielectric.

In a highly preferred embodiment, the low power discharge plasma unit comprises tubular stainless steel gauze electrodes. Whilst various other suitable electrode materials are known in the art, stainless steel is particularly convenient due to inter alia its resistance to corrosion and to oxidative and other damage from the plasma discharge. The purpose of gauze electrodes is to maximize the surface available for the dielectric barrier corona discharge and hence generation of plasma. However, other factors, such as the effects on the electromagnetic field generated, particularly hysteresis effects relating to the generation and collapse of the field during the 50 Hz cycle of the alternating current, also influence the choice of gauze and the fineness of the mesh. In a preferred embodiment the gauze on the outer electrode is coarser than that of the inner electrode as this favours the production of plasma on the outer, rather than inner, electrode. In a more preferred embodiment, the mesh count of the inner electrode is from 50 to 30*45 to 25 (per inch or 25.4 mm) and that of the outer electrode is 35 to 20*40 to 20. In a particularly favoured embodiment, the mesh count of the inner electrode 204 is 40*34 (per inch or 25.4 mm) using a 38 swg wire (0.15 mm diameter) and that of the outer electrode is 24*28 using a 30 swg wire (0.3 mm diameter).

It is also desirable for effective dielectric barrier discharge to take place that the mass of the electrodes be substantially balanced, i.e. to differ by not more than 20%, preferably not more than 10%. This is especially significant in the case of the aforementioned tubular configuration discharge unit.

It will also be appreciated that the power of the dielectric barrier discharge plasma unit is related to the size of the electrodes. In general it is preferred that each of the mesh electrodes should have an area in the range from 25 to 100 cm² preferably from 40 to 90 cm². Typical examples of a coil provided in accordance with the present teaching is one having a length of 5.5 cm or 13 cm and having a diameter of 2.9 cm diameter. Such coils will have exemplary areas of 50 and 118 cm² respectively. The power consumed by the plasma coils is in the range 10 to 20 W resulting in power density of 0.2 to 0.4 W/cm² and 0.08 to 0.16 W/cm² for each coil. Desirably a plasma generator comprising a coil per the present teaching will operate with power density values less than 1 W/cm² and typically in the range from 0.1 to 0.5 W/ cm². It will be appreciated that with a solid dielectric, the generation of a plasma through the use of a dielectric barrier discharge is very much dependent on the thickness of the dielectric, and especially at lower voltages, as used in accordance with the present teachings, it is necessary to minimize the thickness of the dielectric. It will also be understood, though, that the discharge unit must be strong enough to avoid damage by the substantial stresses encountered inside a discharge plasma region.

Figure 5 shows an exploded view of the electrode assembly 24 and holders 48 of figure 4. The inner wire mesh 510 and outer wire mesh 49 are electrically connected to electrode contacts 512. High voltage is applied to holder contacts 513 enabling high voltage of opposite polarities on the inner 510 and outer 49 wire mesh. Power to the drive the electrode assembly is suitably provided by a transformer (not shown). Wiring may be used to provide the voltage from the transformer to the contacts holder 513, which in turn provide the voltage to the electrode contacts 512. The electrode assembly is held together by insulating caps 511. The assembly is supported by the insulating holders 48 and is locked in place on the holders 48 by push rivets 515. The insulating caps 511 and holders 48 ensure that any voltage applied to the holder contacts 513 is not inadvertently applied to the housing of the apparatus. The holders 48 are mounted inside the housing 15 of the apparatus such that the electrode assembly is positioned transverse to the direction of airflow 37. This can also be observed from figure 3.

It should also be noted that the configuration of the inner and outer wire mesh maintaining direct contact around their respective total surface areas of the dielectric glass tube ensures that there are no air pockets around the dielectric assembly 24 where elevated levels of plasma can build up.

The transverse positioning of the electrode assembly 24 relative to the air stream conveying the air to be purified or otherwise treated is also important as it ensures that airflow cannot bypass the electrode assembly without being exposed to plasma discharge. Specifically, the electrode assembly is arranged for generating and discharging plasma within an inactivating zone proximal to the electrode unit. The present inventors make use of an understanding that an inactivation efficacy of said plasma discharge is dependent on the air flowing in the vicinity of the plasma discharge and therefore arranging the electrode assembly transverse to the direction of the airflow ensures that an inactivating zone is created around the electrode unit 24 that the airflow 37 must pass through in order to reach the outlet regions 12.

In accordance with the present teaching, the mounting of the electrode assembly 24 away from the internal surface of the housing 15 of the apparatus is also beneficial. Such mounting may be provided by use of holders 48 which ensure maximum efficiency of the electrode assembly 24. Plasma is discharged from the outer mesh 49 to create the aforementioned inactivation zone. By having the electrode assembly raised, this inactivation zone extends completely around the electrode assembly and air can pass both above and below the electrode assembly 24 while being subjected to the same level of plasma.

The plasma concentration in the inactivating zone, created by the electrode assembly, is sufficient to effectively inactivate airborne pollutant material entrained in the air flow. Furthermore the concentration of plasma decays sufficiently outside the inactivating zone so that the concentration of any anti-pathogenic agents created by the plasma discharge in the cleaned air expelled from the outlet regions 12 of the apparatus is at a physiologically acceptable level.

It should be noted that, as described above, the method and apparatus of the present invention does not include the use of physical air filtration system, such as pre-filter, HEPA filters or equivalent.

In an alternative embodiment of the apparatus, air is directed or forced around the plasma discharge through a ducting system. Said ducting system is designed to ensure that all air flow about the plasma discharge within 1 centimetre of the discharge.

Exemplary testing of an apparatus provided in accordance with the present teaching showed efficacy in killing both bacteria and virus. Table 1 shows exemplary data for bacteria testing whereas Table 2 shows similar results for viral testing.

**Table 1**

| | | | | | |
|---|---|---|---|---|---|
| Escherichia coli | Gram -ve | 2.1E+05 | 0.0E+00 | >5 | >99.999 |
| S.tyhpi murium | Gram -ve | 4.6E+05 | 0.0E+00 | >5 | >99.999 |
| E. agglormerans | Gram -ve | 3.9E+05 | 0.0E+00 | >5 | >99.999 |
| E.gergoviae | Gram -ve | 4.2E+05 | 0.0E+00 | >5 | >99.999 |
| A.aerogens | Gram -ve | 7.1E+05 | 0.0E+00 | >5 | >99.999 |
| S.marcescens | Gram -ve | 8.2E+05 | 0.0E+00 | >5 | >99.999 |
| E.sakazakii | Gram -ve | 3.4E+05 | 0.0E+00 | >5 | >99.999 |
| Ecoli 0157 H:7 | Gram -ve | 3.5E+05 | 0.0E+00 | >5 | >99.999 |
| P.aeruginosa | Gram -ve | 6.1E+05 | 0.0E+00 | >5 | >99.999 |
| P.putida | Gram -ve | 8.2E+05 | 0.0E+00 | >5 | >99.999 |
| | | | | | |
| S-aureus oxford | Gram +ve | 4.3E+05 | 0.0E+00 | >5 | >99.999 |
| S.aureus MSRA | Gram +ve | 4.8E+05 | 0.0E+00 | >5 | >99.999 |
| S.epidermidis | Gram +ve | 3.7E+05 | 0.0E+00 | >5 | >99.999 |
| M.luteus | Gram +ve | 9.0E+05 | 0.0E+00 | >5 | >99.999 |
| S.faecalis | Gram +ve | 7.3E+05 | 0.0E+00 | >5 | >99.999 |
| S.pyogenes | Gram +ve | 3.6E+05 | 0.0E+00 | >5 | >99.999 |
| B-coreus | Gram +ve | 7.1E+05 | 0.0E+00 | >5 | >99.999 |
| B.globigii | G+ve Spore | 7.9E+05 | 1.0E+01 | >5 | 99.999 |
| B.subtilis | G+ve Spore | 2.1E+05 | 3.0E+01 | >5 | 99.986 |
| B. megaterium | G+ve Spore | 6.2E+05 | 9.0E+01 | >5 | 99.985 |
| | | | | | |
| S.cerevisiea | Yeast | 4.3E+05 | 0.0E+00 | >5 | >99.999 |
| S.bailli | Yeast | 7.2E+05 | 0.0E+00 | >5 | >99.999 |
| Pichia mixed sps | Yeast | 6.3E+05 | 0.0E+00 | >5 | >99.999 |
| S.ludwigii | Yeast | 6.0E+05 | 0.0E+00 | >5 | >99.999 |
| A.niger | Mould mycelial | 6.2E+05 | 0.0E+00 | >5 | >99.999 |
| A.flavus | Mould mycelial | 7.8E+05 | 0.0E+00 | >5 | >99.999 |
| F.poea | Mould mycelial | 7.2E+05 | 0.0E+00 | >5 | >99.999 |
| P.digitatum | Mould mycelial | 6.9E+05 | 0.0E+00 | >5 | >99.999 |
| F graminerium | Mould mycelial | 4.3E+05 | 0.0E+00 | >5 | >99.999 |
| A.niger | Mould Spore | 8.2E+05 | 7.0E+01 | >5 | 99.991 |
| A.flavus | Mould Spore | 6.7E+05 | 5.0E+01 | >5 | 99.993 |
| F.poea | Mould Spore | 8.2E+05 | 0.0E+00 | >5 | >99.999 |
| P.digitatum | Mould Spore | 6.7E+05 | 0.0E+00 | >5 | >99.999 |
| F gramenerium | Mould Spore | 2.9E+05 | 0.0E+00 | >5 | >99.999 |

**Table 2**

| Organism | Class | Mean cfu/m³/Hr at input Treatment stream | Mean cfu/m³/Hr post Treatment exit stream | Mean decline Log/cfu/m³/Hr post Treatment exit stream | Apparent percentage reduction |
|---|---|---|---|---|---|
| CTX | SS DNA | 4.3E+12 | 8.1E+02 | >12 | >99.999 |
| ScV-L-BC | DS RNA | 9.2E+12 | 4.6E+02 | >12 | >99.999 |
| FcoV (attenuated) | SS + RNA | 7.1E+12 | 3.0E+02 | >12 | >99.999 |
| T4 Phage | DS DNA | 5.3E+12 | 7.4E+02 | >12 | >99.999 |

In both examples of Table 1 and Table 2, a device per the present teaching with the plasma generator operating at power densities less than 1 W/cm² achieved >Log 5 kill rate for all classes of bacteria or viral matter.

Situations in which the anti-microbial applications of the invention are especially useful include hospitals, food preparation areas, laboratories and locations with limited ventilation, where air may be re-circulated. Storage of sterile instruments and materials in an atmosphere sterilised by means of the invention may extend their shelf life, with considerable consequent savings. The invention provides a means of supplying a unit for such storage with sterile, dry air capable of maintaining the sterility of stored instruments for extended periods. One particularly useful application is in flood-damaged buildings, where removal of fungal spores from the air can minimize subsequent growth of mould and development of rot in the fabric of the building, with significant reduction in damage and costs of repair. In another application, the apparatus may be installed in ducting or pipework carrying a flow of air, such as may for example be used in an air-conditioning system.

It will be appreciated that in general where airborne pollutants or pathogens are being removed from a room or other more or less enclosed space, the amount of treatment required will depend on the nature of the pollutant/pathogen, and possibly also the burden or loading thereof in the air. Whilst there may in principle be used multiple passes to progressively reduce the pollutant/pathogen loading, it is a particular advantage of the invention that the relatively high anti-pathogenic agent concentrations which can be achieved with apparatus of the invention within the contained inactivation zone, can usually provide substantially complete inactivation within a single pass, thereby minimizing the number of air changes required. Typically where it is desired to remove bacterial pollutants there should be provided at least 5 air changes per hour, whilst in the case of locations with moderate to high tobacco smoke loadings, it may be desirable to provide at least 10 or more air changes. The total airflow required to treat a room may be readily determined from the volume of the room and the number of air changes required. Whilst it might in principle be possible to achieve higher flow rates with larger sizes of apparatus, it is generally preferred to achieve them by using multiple apparatus units. In this connection it will be appreciated that more than one discharge plasma unit may be mounted in the same conduit, provided the inactivation zones of all the generators are contained within the conduit. Furthermore, more than one discharge unit may be powered by one (common) transformer, albeit the total power of the transformer will then be divided between the discharge units.

The words comprises/comprising when used in this specification are to specify the presence of stated features, integers, steps or components but does not preclude the presence or addition of one or more other features, integers , steps, components or groups thereof.

## Claims

1. An air treatment apparatus comprising:
a housing (15) defining an air flow path between an inlet (11) and an outlet (12); an air flow impeller (23) arranged for inducing a flow of air through said air flow path; and
an electrode assembly (24) suspended within the air flow path and orientated transverse to the induced air flow path; wherein the electrode assembly (24) is configured such that air is forced into the apparatus via the inlet (11), forced around the electrode assembly (24) and expelled through the outlet (12) whereby the air is passed over and under the electrode assembly (24) and not passed through the electrode assembly (24); wherein the electrode assembly (24) is configured to operate at a power density less than 1 W/cm² to operably generate a plasma discharge circumferentially about a longitudinal axis of the electrode assembly (24) and within which particles or constituents of air within the induced air flow will be exposed to the plasma.

2. The air treatment apparatus of claim 1 wherein the electrode assembly is cylindrically shaped having a length greater than its diameter, the electrode assembly being orientated within the housing such that its longitudinal axis is provided transverse to the induced air flow path.

3. The air treatment apparatus of claim 2 further comprising a holder at each end of the electrode assembly for mounting the electrode assembly in the elevated position away from side walls of housing; optionally wherein the holders comprise an insulating material for preventing conduction between the electrode assembly and the housing.

4. The air treatment apparatus of claim 2 wherein the electrode assembly includes a cylindrical inner mesh and an outer mesh separated by a cylindrical dielectric barrier.

5. The air treatment apparatus of claim 4 wherein the electrode assembly includes an insulating cap at each end of the electrode assembly for holding the inner mesh, outer mesh and dielectric barrier in contact with each other.

6. The air treatment apparatus of claim 4 or 5 wherein a respective surface of the inner mesh and an outer mesh is in complete contact with the cylindrical dielectric barrier.

7. The air treatment apparatus of any one of claims 4 to 6 wherein the inner mesh and outer mesh are electrically connected to electrode contacts at each end of the electrode assembly, the contacts providing voltage of opposite polarities to the inner and outer meshes.

8. The air treatment apparatus of any one of claims 4 to 7 wherein at least one of the inner mesh and the outer mesh extend completely about the electrode assembly.

9. The air treatment apparatus of claim 8 wherein at least one of the inner mesh and mesh provide a continuous surface about a longitudinal axis of the electrode assembly.

10. The air treatment apparatus of any preceding claim wherein the housing comprising a ducting system for directing the air flow path to and about the electrode assembly;
optionally wherein the housing is rectangular shaped with the inlet at a first end and the outlet at a second end; and optionally, wherein the outlet includes an outlet region on each of two adjacent sides of the housing.

11. The air treatment apparatus of any preceding claim wherein the air exits the outlet transverse to the air flow path.

12. The air treatment apparatus of any preceding claim wherein the power supply is operated at a power density in the range from 0.1 to 0.5 W/cm².

13. The air treatment apparatus of any preceding claim wherein the plasma generator comprises concentric tubular metal gauze electrodes separated by a dielectric;
Optionally wherein the electrodes comprise a mesh.

14. The air treatment apparatus of claim 13 wherein an inner and outer electrode are provided and the gauze on the outer electrode is coarser than that of the inner electrode to favour production of plasma on the outer, rather than inner, electrode;
optionally wherein the inner electrode has a mesh count of 40*34 per 25.4 mm using a wire of 0.15 mm diameter and the outer electrode has mesh count of 24*28 per 25.4 mm using a wire of diameter 0.3 mm.

15. The air treatment apparatus of any preceding claim wherein the apparatus is configured such that all air flow about the plasma discharge is directed within 1 centimetre of the discharge.

## Patentansprüche

1. Luftbehandlungsvorrichtung, die umfasst:
ein Gehäuse (15), das einen Luftströmungsweg zwischen einem Einlass (11) und einem Auslass (12) definiert;
ein Luftstromflügelrad (23), das so ausgelegt ist, dass es einen Luftstrom durch den Luftströmungsweg induziert; und
eine Elektrodenbaugruppe (24), die innerhalb des Luftströmungswegs aufgehängt und quer zum induzierten Luftströmungsweg ausgerichtet ist; wobei die Elektrodenbaugruppe (24) so konfiguriert ist, dass Luft über den Einlass (11) in die Vorrichtung gezwungen wird, um die Elektrodenbaugruppe (24) gezwungen wird und durch den Auslass ausgestoßen wird, wodurch die Luft über und unter der Elektrodenbaugruppe (24) passiert und nicht durch die Elektrodenbaugruppe (24) passiert;
wobei die Elektrodenbaugruppe (24) so konfiguriert ist, dass sie mit einer Leistungsdichte von weniger als 1 W/cm² arbeitet, um eine Plasmaentladung umfangsmäßig um eine Längsachse der Elektrodenbaugruppe (24) funktionsmäßig zu erzeugen und innerhalb welcher Partikel oder Bestandteile von Luft innerhalb des induzierten Luftstroms dem Plasma ausgesetzt werden.

2. Luftbehandlungsvorrichtung nach Anspruch 1, wobei die Elektrodenbaugruppe zylinderförmig ist, wobei eine Länge größer als deren Durchmesser ist, wobei die Elektrodenbaugruppe innerhalb des Gehäuses ausgerichtet ist, so dass ihre Längsachse quer zum induzierten Luftströmungsweg angeordnet ist.

3. Luftbehandlungsvorrichtung nach Anspruch 2, die ferner eine Halterung an jedem Ende der Elektrodenbaugruppe zum Anbringen der Elektrodenbaugruppe in der erhöhten Position weg von Seitenwänden eines Gehäuses umfasst; optional wobei die Halterungen ein Isoliermaterial umfassen, um ein Leiten zwischen der Elektrodenbaugruppe und dem Gehäuse zu verhindern.

4. Luftbehandlungsvorrichtung nach Anspruch 2, wobei die Elektrodenbaugruppe ein zylindrisches Innennetz und ein Außennetz umfasst, die durch eine zylindrische dielektrische Barriere getrennt sind.

5. Luftbehandlungsvorrichtung nach Anspruch 4, wobei die Elektrodenbaugruppe eine Isolierkappe an jedem Ende der Elektrodenbaugruppe umfasst, um das Innennetz, das Außennetz und die dielektrische Barriere miteinander in Kontakt zu halten.

6. Luftbehandlungsvorrichtung nach Anspruch 4 oder 5, wobei eine jeweilige Oberfläche des Innennetzes und eines Außennetzes mit der zylindrischen dielektrischen Barriere in vollständigen Kontakt steht.

7. Luftbehandlungsvorrichtung nach einem der Ansprüche 4 bis 6, wobei das Innennetz und das Außennetz an jedem Ende der Elektrodenbaugruppe mit Elektrodenkontakten elektrisch verbunden sind, wobei die Kontakte Spannung entgegengesetzter Polaritäten an die Innen- und Außennetze bereitstellen.

8. Luftbehandlungsvorrichtung nach einem der Ansprüche 4 bis 7, wobei zumindest eines des Innennetzes und des Außennetzes sich vollständig um die Elektrodenbaugruppe erstreckt.

9. Luftbehandlungsvorrichtung nach Anspruch 8, wobei zumindest eines des Innennetzes und Netzes eine durchgehende Fläche um eine Längsachse der Elektrodenbaugruppe bereitstellt.

10. Luftbehandlungsvorrichtung nach einem vorstehenden Anspruch, wobei das Gehäuse ein Kanalsystem zum Leiten des Luftströmungswegs zu und um die Elektrodenbaugruppe umfasst;
optional wobei das Gehäuse rechteckig ist, wobei sich der Einlass an einem ersten Ende befindet und sich der Auslass an einem zweiten Ende befindet; und optional wobei der Auslass einen Auslassbereich an jeder von zwei benachbarten Seiten des Gehäuses umfasst.

11. Luftbehandlungsvorrichtung nach einem vorstehenden Anspruch, wobei die Luft quer zum Luftströmungsweg aus dem Auslass austritt.

12. Luftbehandlungsvorrichtung nach einem vorstehenden Anspruch, wobei die Energieversorgung bei einer Leistungsdichte im Bereich von 0,1 bis 0,5 W/cm² betrieben wird.

13. Luftbehandlungsvorrichtung nach einem vorstehenden Anspruch, wobei der Plasmagenerator konzentrische rohrförmige Metallgewebeelektroden umfasst, die durch ein Dielektrikum getrennt sind;
optional wobei die Elektroden ein Netz umfassen.

14. Luftbehandlungsvorrichtung nach Anspruch 13, wobei eine Innen- und eine Außenelektrode bereitgestellt sind und das Gewebe auf der Außenelektrode gröber als jenes der Innenelektrode ist, um die Erzeugung von Plasma auf der Außenelektrode gegenüber der Innenelektrode zu begünstigen; optional wobei die Innenelektrode eine Maschenweite von 40 x 34 pro 25,4 mm bei Verwendung eines Drahts mit einem Durchmesser von 0,15 mm aufweist und die Außenelektrode eine Maschenweite von 24 x 28 pro 25,4 mm bei Verwendung eines Drahts mit einem Durchmesser von 0,3 mm aufweist.

15. Luftbehandlungsvorrichtung nach einem vorstehenden Anspruch, wobei die Vorrichtung so konfiguriert ist, dass der gesamte Luftstrom um die Plasmaentladung innerhalb eines Zentimeters der Entladung geleitet wird.

## Revendications

1. Appareil de traitement de l'air comprenant :
un boîtier (15) définissant un trajet d'écoulement d'air entre une entrée (11) et une sortie (12) ;
une roue à aubes d'écoulement d'air (23) agencée pour induire un écoulement d'air à travers ledit trajet d'écoulement d'air ; et
un ensemble électrode (24) suspendu à l'intérieur dudit trajet d'écoulement d'air et orienté transversalement au trajet d'écoulement d'air induit ; l'ensemble électrode (24) étant configuré de telle sorte que de l'air est forcé dans l'appareil par l'intermédiaire de l'entrée (11), forcé autour de l'ensemble électrode (24) et expulsé à travers la sortie (12), ce par quoi l'air est amené à passer sur et sous l'ensemble électrode (24), et à ne pas passer à travers l'ensemble électrode (24) ;
l'ensemble électrode (24) étant configuré pour fonctionner à une densité de puissance de moins de 1 W/cm² afin de générer, de manière fonctionnelle, une décharge de plasma de manière circonférentielle autour d'un axe longitudinal de l'ensemble électrode (24), et dans laquelle des particules ou des constituants de l'air dans l'écoulement d'air induit seront exposés au plasma.

2. Appareil de traitement de l'air selon la revendication 1, dans lequel l'ensemble électrode a une forme cylindrique ayant une longueur plus grande que son diamètre, l'ensemble électrode étant orienté à l'intérieur du boîtier de telle sorte que son axe longitudinal est disposé transversalement au trajet d'écoulement d'air induit.

3. Appareil de traitement de l'air selon la revendication 2, comprenant en outre un support à chaque extrémité de l'ensemble électrode pour monter l'ensemble électrode dans la position surélevée à l'écart des parois latérales du boîtier ; facultativement, les supports comprenant un matériau isolant pour empêcher une conduction entre l'ensemble électrode et le boîtier.

4. Appareil de traitement de l'air selon la revendication 2, dans lequel l'ensemble électrode comprend un treillis interne cylindrique et un treillis externe séparés par une barrière diélectrique cylindrique.

5. Appareil de traitement de l'air selon la revendication 4, dans lequel l'ensemble électrode comprend un capuchon isolant à chaque extrémité de l'ensemble électrode pour maintenir le treillis interne, le treillis externe et la barrière diélectrique en contact les unes avec les autres.

6. Appareil de traitement de l'air selon la revendication 4 ou 5, dans lequel une surface respective du treillis interne et du treillis externe est en contact complet avec la barrière diélectrique cylindrique.

7. Appareil de traitement de l'air selon l'une quelconque des revendications 4 à 6, dans lequel le treillis interne et le treillis externe sont reliées électriquement à des contacts d'électrode au niveau de chaque extrémité de l'ensemble électrode, les contacts fournissant une tension de polarités opposées aux treillis interne et externe.

8. Appareil de traitement de l'air selon l'une quelconque des revendications 4 à 7, dans lequel au moins un parmi le treillis interne et le treillis externe s'étend entièrement autour de l'ensemble électrode.

9. Appareil de traitement de l'air selon la revendication 8, dans lequel au moins un parmi le treillis interne et le treillis externe fournit une surface continue autour d'un axe longitudinal de l'ensemble électrode.

10. Appareil de traitement de l'air selon l'une quelconque des revendications précédentes, dans lequel le boîtier comprend un système de conduit pour diriger le trajet d'écoulement d'air vers et autour de l'ensemble électrode ; facultativement, le boîtier étant de forme rectangulaire avec l'entrée à une première extrémité et la sortie à une seconde extrémité ; et, facultativement, la sortie comprenant une région de sortie sur chacun des deux côtés adjacents du boîtier.

11. Appareil de traitement de l'air selon l'une quelconque des revendications précédentes, dans lequel l'air sort de la sortie transversalement au trajet d'écoulement d'air.

12. Appareil de traitement de l'air selon l'une quelconque des revendications précédentes, dans lequel l'alimentation électrique fonctionne à une densité de puissance comprise dans la plage allant de 0,1 à 0,5 W/cm².

13. Appareil de traitement de l'air selon l'une quelconque des revendications précédentes, dans lequel le générateur de plasma comprend des électrodes à grille métalliques tubulaires concentriques séparées par un diélectrique ; facultativement, les électrodes comprennent un treillis.

14. Appareil de traitement de l'air selon la revendication 13, dans lequel des électrodes interne et externe sont prévues, et la grille sur l'électrode externe est plus grossière que celle de l'électrode interne pour favoriser la production de plasma sur l'électrode externe, plutôt que sur l'électrode interne ;
facultativement, l'électrode interne a un maillage de 40*34 par 25,4 mm en utilisant un fil de 0,15 mm de diamètre et l'électrode externe a un maillage de 24*28 par 25,4 mm en utilisant un fil de 0,3 mm de diamètre.

15. Appareil de traitement de l'air selon l'une quelconque des revendications précédentes, dans lequel l'appareil est configuré de telle sorte que la totalité de l'écoulement d'air autour de la décharge de plasma est dirigée dans les limites d'1 centimètre de la décharge.
